(19) Europäisches Patentamt
European Patent Office
Office européen des brevets

(11) EP 0 529 373 B1

(12) EUROPÄISCHE PATENTSCHRIFT

(45) Veröffentlichungstag und Bekanntmachung des Hinweises auf die Patenterteilung:
**18.06.1997 Patentblatt 1997/25**

(21) Anmeldenummer: **92113508.3**

(22) Anmeldetag: **07.08.1992**

(51) Int Cl.[6]: **C07C 323/03**, C07C 323/12, C23F 11/16, C07C 323/25, C07C 323/52, C07C 323/66, C07D 209/48

(54) **Disulfide, Verfahren zu deren Herstellung und deren Verwendung**

Disulfides, process for their preparation and their use

Disulfures, procédé pour leur préparation et leur utilisation

(84) Benannte Vertragsstaaten:
**AT BE CH DE DK ES FR GB IT LI NL SE**

(30) Priorität: **23.08.1991 DE 4127821**

(43) Veröffentlichungstag der Anmeldung:
**03.03.1993 Patentblatt 1993/09**

(73) Patentinhaber: **BASF Aktiengesellschaft
67063 Ludwigshafen (DE)**

(72) Erfinder:
- **Keller, Harald, Dr.
W-6700 Ludwigshafen (DE)**
- **Schrepp, Wolfgang, Dr.
W-6900 Heidelberg (DE)**
- **Fuchs, Harald, Dr.
W-6719 Carlsberg (DE)**

(56) Entgegenhaltungen:
- **CHEMICAL ABSTRACTS, Band 110, Nr. 3, 16. Januar 1989, Seite 526, Spalten 1-2, Zusammenfassung Nr. 23626n, Columbus, Ohio, US; & JP-A-63 51 386 (KYOWA HAKKO KOGYO CO., LTD) 04-03-1988**
- **JOURNAL OF THE AMERICAN CHEMICAL SOCIETY, Band 109, Nr. 8, 15. April 1987, Seiten 2358-2368, American Chemical Society; R.G. NUZZO et al.: "Spontaneously organized molecular assemblies. 3. Preparation and properties of solution adsorbed monolayers of organic disulfides on gold surfaces"**
- **JOURNAL OF THE AMERICAN CHEMICAL SOCIETY, Band 112, Nr. 2, 17. Januar 1990, Seiten 558-569, American Chemical Society; R.G. NUZZO et al.: "Fundamental studies of microscopic wetting on organic surfaces. 1. Formation and structural characterization of a self-consistent series of polyfunctional organic monolayers"**
- **JOURNAL OF THE AMERICAN CHEMICAL SOCIETY, Band 111, Nr. 1, 4. Januar 1989, Seiten 321-335, American Chemical Society; C.D. BAIN et al.: "Formation of monolayer films by the spontaneous assembly of organic thiols from solution onto gold"**
- **CHEMICAL ABSTRACTS, Band 108, Nr. 4, 25. Januar 1988, Seite 364, Spalte 1, Zusammenfassung Nr. 27375m, Columbus, Ohio, US; L.H. DUBOIS et al.: "Fundamental studies of the interactions of adsorbates on organic surfaces", & PROC. NATL. ACAD. SCI. USA 1987, 84(14), 4739-42**
- **Proc.Natl.Acad.Sci.USA 84,4739-4742**
- **Langmuir 5(1),101-111(1989)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 529 373 B1

Printed by Jouve, 75001 PARIS (FR)

## Beschreibung

Die Erfindung betrifft neue organische Disulfide, deren Herstellung sowie die Herstellung von chemisorbierten Disulfidfilmen auf Edelmetallträgern.

Modifizierung von Trägeroberflächen ist in der industriellen Technik weit verbreitet. Der Schutz vor Abrieb oder Ausbleichen und der Korrosionsschutz seien beispielhaft für den Materialschutz durch spezielle Beschichtungen erwähnt. In der optischen Industrie finden Oberflächenmodifizierungen als Frequenzfilter und Reflektoren weite Verwendung. Modifzierung von Oberflächen auf molekularer Ebene, beispielsweise durch Aufbringen von monomolekularen Schichten, ist erst in den letzten Jahren intensiver untersucht und entwickelt worden. So ist bekannt, daß organische Disulfide und Thiole aus Lösung spontan auf Gold- und Silberoberflächen chemisorbieren. Nach Abspülen mit Lösungsmittel ergeben sich stabile monomolekulare Filme auf dem Metallsubstrat. Endständige funktionelle Gruppen der Disulfid- bzw. Thiolatfilme beeinflussen die Benetzbarkeit der beschichteten Metalloberfläche (vgl. C.D. Bain, E. B. Troughton, Y.T. Tao, J. Evall, G.M. Whitesides, R.G. Nuzzo, J. Am. Chem. Soc. 111, 321 (1989), C.J. Sandroff, D. R. Hershbach, J. Phys. Chem. 86, 2700 (1982), C.D. Bain, G.M. Whitesides, Angew. Chem. 101, 522 (1989)).

Organische Disulfide mit endständigen funktionellen Gruppen sind bekannt, jedoch wurden in der Vergangenheit vor allem kurzkettige Disulfide mit endständigen funktionellen Gruppen synthetisiert. Disulfide können auf verschiedenen Wegen synthetisiert werden, wie z.B. durch Oxidation von Thiolen, nukleophile Substitution mittels $(S\text{-}S)^{2-}$ und Oxidation von Buntesalzen (vgl. E. Müller, Methoden der Organischen Chemie, Houben-Weyl, Bd. 9, 55-82 (1955)).

Kurzkettige Disulfide haben den Nachteil, daß sie als monomolekulare Filme keine Möglichkeit zum zweidimensionalen Kristallisieren haben. Dadurch sind solche Filme nicht dicht und weisen auch keinen hohen Ordnungsgrad auf. Außerdem ist die mechanische und thermische Stabilität kurzkettiger Disulfidfilme geringer als die Filme von langkettigen Disulfiden.

In Proc. Natl. Acad. Sci. USA 84 (1987), 4739-4742 sind funktionalisierte Dialkyldisulfidderivate zur monomolekularen Beschichtung von Gold durch Chemisorption beschrieben, wobei auch von entsprechenden Alkylthiolen ausgegangen werden kann. In J. Am. Chem. Soc. 112 (1990), 558-569 wird die Möglichkeit aufgezeigt, längerkettige Alkylthiole mit endständigen funktionellen Gruppen an Goldoberflächen zu adsorbieren.

Die Publikation von N. Tillman, A. Ulman und Th. L. Penner in Langmuir, The ACS Journal of Surfaces and Colloids, Vol. 5, No. 1 (1989), 101 bis 111 bezieht sich auf die Bildung von Ein- und Mehrfachschichten durch Self-Assembly, wobei auch 11-Hydroxy-1-undecanthiol zur Beschichtung von Gold und Silber aufgeführt wird.

Aufgabe der vorliegenden Erfindung ist es, Disulfide aufzuzeigen, die reproduzierbar herzustellen sind und folgende Vorteile aufweisen:

a) Als monomolekulare chemisorbierte Schichten sollen sie sehr dichte Filme bilden und dadurch Korrosionsschutz gewährleisten.

b) Als monomolekulare chemisorbierte Schichten sollen sie endständige funktionelle Gruppen in hohem Ausmaß an der Oberfläche exponieren und dadurch das chemische Anbinden weiterer Schichten, sowie das gezielte Modifizieren von Benetzungseigenschaften ermöglichen.

Überraschenderweise läßt sich dies mit den erfindungsgemäßen langkettigen Disulfiden erreichen.

Gegenstand der vorliegenden Erfindung sind Disulfide der allgemeinen Formel (I)

$$X^1\text{-}(CH_2)_n\text{-}S\text{-}S(CH_2)_n\text{-}X^2 \qquad \text{(I)},$$

worin $X^1$ und $X^2$ untereinander gleich sind und für die Substituenten
-N-Phthalimid, $-NH_2$, $-OOC-(CH_2)_3-COOH$, $-OSO_2-CH_3$,
$-NH-(CH_2)_2-NH_2$, $-SO_3H$ oder $-SO_3^-M^+$ mit $M^+ = Li^+$, $Na^+$ oder $K^+$ stehen und n für eine ganze Zahl von 11 bis 25 steht, oder $X^1$ für -OH und $X^2$ für -Br stehen und n für 11 bis 25 steht.

Bevorzugt sind Disulfide der allgemeinen Formel (I), worin n = 11 bis 13 bedeutet.

Gegenstand der vorliegenden Erfindung sind auch folgende Verfahren zur Herstellung der erfindungsgemäßen Disulfide, nämlich ein Verfahren zur Herstellung der Disulfide der allgemeinen Formel (I) $X^1\text{-}(CH_2)_n\text{-}S\text{-}S\text{-}(CH_2)_n\text{-}X^2$, worin die Substituenten $X^1$ und $X^2$ untereinander gleich sind und für $-OOC-(CH_2)_3-COOH$ oder $-OSO_2-CH_3$ stehen oder $X^1$ für -OH und $X^2$ für -Br steht, das dadurch gekennzeichnet ist, daß im ersten Syntheseschritt ein endständig bromsubstituierter Alkohol der Formel $Br\text{-}(CH_2)_n\text{-}OH$ mit n = 11 bis 25 mit der äquimolaren Menge Natriumthiosulfat in Wasser oder einem polaren organischen Lösungsmittel bei Temperaturen von 60 bis 120°C

zum Buntesalz umgesetzt wird, im zweiten Syntheseschritt die Lösung des Buntesalzes mit Iod zum Disulfid oxidiert wird, im dritten Syntheseschritt das Disulfid mit Bromwasserstoff bei 60 bis 120°C behandelt wird, wobei sowohl das Monosubstitutionsprodukt ($X^1$ = -OH, $X^2$ = -Br) als auch das Disubstitutionsprodukt ($X^1$ = $X^2$ = -Br) gebildet wird, und die beiden unterschiedlich substituierten Disulfide getrennt werden, wobei für den Fall, daß $X^1$ und $X^2$ in der Formel (I) für $-OOC-(CH_2)_3-COOH$ stehen, das Produkt des 2. Syntheseschrittes im dritten Syntheseschritt mit Glutarsäureanhydrid bei 70 bis 120°C zum entsprechenden Halbester der Glutarsäure umgesetzt wird und gegebenenfalls anschließend durch Kristallisation gereinigt wird, für den Fall, daß $X^1$ und $X^2$ in der Formel (I) für $-OSO_2-CH_3$ stehen, das Produkt des 2. Syntheseschrittes im dritten Syntheseschritt bei Temperaturen zwishen 0 bis 20°C in einem wasserfreien aprotischen organischen Lösungsmittel mit Methansulfonylchlorid in Gegenwart eines tertiären Amins umgesetzt wird, das entstandene Aminhydrochlorid entfernt, und aus der verbleibenden Lösung nach Verdampfen des Lösungsmittels das Disulfid mit $X^1$ = $X^2$ = $-O-SO_2-CH_3$ erhalten wird;

ebenso ein Verfahren zur Herstellung der Disulfide der allgemeinen Formel (I), worin die Substituenten $X^1$ und $X^2$ untereinander gleich sind und für $-NH-(CH_2)_2-NH_2$ stehen, das dadurch gekennzeichnet ist, daß das nach dem vorstehenden Verfahren hergestellte Disulfid mit $X^1$ = $X^2$ = $-O-SO_2-CH_3$ mit überschüssigem Ethylendiamin bei 50 bis 100°C umgesetzt und gegebenenfalls gereinigt wird;

ferner ein Verfahren zur Herstellung der Disulfide der allgemeinen Formel (I), worin die Substituenten $X^1$ und $X^2$ untereinander gleich sind und für -N-Phthalimid stehen, das dadurch gekennzeichnet ist, daß in einem ersten Syntheseschritt ein endständig bromsubstituierter Alkohol der Formel $Br-(CH_2)_n-OH$ mit n = 11 bis 25 mit der äquimolaren Menge Kaliumphthalimid in einem polaren aprotischen Lösungsmittel bei 50 bis 100°C umgesetzt, das Reaktionsprodukt durch Ausfällen in Wasser erhalten und gegebenenfalls durch Umkristallisieren gereinigt wird, das so erhaltene Produkt in einem zweiten Syntheseschritt mit einem Überschuß an konzentrierter wäßriger Bromwasserstofflösung und konzentrierter Schwefelsäure auf Temperaturen zwischen 60 und 120°C erhitzt, das bei Raumtemperatur ausfallende Produkt,
gegebenenfalls durch Umkristallisieren gereinigt, in einem dritten Syntheseschritt mit der äquimolaren Menge Natriumthiosulfat versetzt und in Wasser oder einem polaren organischen Lösungsmittel oder dessen Gemisch mit Wasser auf Temperaturen von 60 bis 120°C erhitzt, das so erhaltene Produkt, gegebenenfalls durch Umkristallisieren gereinigt, in einem vierten Syntheseschritt in einem protischen organischen Lösungsmittel gelöst und mit Iod oxidiert und gegebenenfalls durch Umkristallisieren gereinigt wird.

Das Verfahren zur Herstellung der Disulfide der allgemeinen Formel (I), worin die Substituenten $X^1$ und $X^2$ untereinander gleich sind und für $-NH_2$ stehen, ist dadurch gekennzeichnet, daß das gemäß dem vorstehenden Verfahren hergestellte Produkt mit überschüssigem Hydrazin in einem polaren organischen Lösungsmittel bei Raumtemperatur behandelt wird, der dabei entstandene Niederschlag durch Zugabe von wäßriger Alkalilauge in Lösung gebracht, das Produkt mit Ether extrahiert und das nach Verdampfen des Ethers erhaltene Rohprodukt durch Umkristallisieren gereinigt wird.

Das Verfahren zur Herstellung der Disulfide der allgemeinen Formel (I), worin die Substituenten $X^1$ und $X^2$ untereinander gleich sind und für $-SO_3^-M^+$, z.B. $-SO_3^-Na^+$, stehen, ist dadurch gekennzeichnet, daß in einem ersten Syntheseschritt ein entständig bromsubstituierter Alkohol der Formel $Br-(CH_2)_n-OH$ mit n = 11 bis 25 mit der in Wasser gelösten äquimolaren Menge Natriumsulfit bei Temperaturen zwishen 80 und 120°C umgesetzt, das beim Abkühlen anfallende, gegebenenfalls umkristallisierte, Produkt in einem zweiten Syntheseschritt mit einem Überschuß einer wäßrigen konzentrierten Bromwasserstofflösung und konzentrierter Schwefelsäure auf Temperaturen zwischen 60 und 120°C erhitzt, das beim Abkühlen ausfallende, gegebenenfalls umkristallisierte, Produkt in einem dritten Syntheseschritt mit der äquimolaren Menge Natriumthiosulfat in Wasser bei Temperaturen zwischen 80 und 120°C umgesetzt wird, zur siedenden Lösung dieses Umsetzungsproduktes die halbmolare Menge Iod, gelöst in einem polaren organischen Lösungsmittel, getropft, anschließend mit Natriumcarbonat neutralisiert und das beim Abkühlen ausfallende Produkt gegebenenfalls aus Wasser umkristallisiert wird.

Disulfide der allgemeinen Formel (I), worin die Substituenten $X^1$ und $X^2$ untereinander gleich sind und für $-SO_3H$ stehen, werden dadurch erhalten, daß Disulfide mit $-SO_3Na$-Gruppen, die gemäß dem vorstehenden Verfahren erhältlich sind, in überschüssiger konzentrierter Mineralsäure aufgekocht und der beim Abkühlen ausfallende Feststoff gegebenenfalls aus Wasser umkristallisiert wird.

Gegenstand der vorliegenden Erfindung ist auch ein Verfahren zur Herstellung monomolekularer Schichten auf Edelmetallträgern mit den erfindungsgemäßen Disulfiden, das dadurch gekennzeichnet ist , daß der Edelmetallträger mit einer Lösung des erfindungsgemäßen Disulfides in Kontakt gebracht wird, wodurch eine Chemisorption des Disulfides auf dem Edelmetallträger erfolgt.

Gegenstand der vorliegenden Erfindung ist außerdem ein Verfahren zur Herstellung von multimolekularen Schichten auf Trägern, deren Oberfläche durch Aufbringen von erfindungsgemäßen Disulfidschichten nach dem vorstehenden Verfahren modifiziert ist, wobei der Aufbau der multimolekularen Schichten sequenziell, d.h. Schicht für Schicht, erfolgt und die einzelnen Schichten miteinander chemisch verbunden sind.

Die Haftung der erfindungsgemäßen Disulfide auf dem Edelmetallträger erfolgt durch kovalente Bindung über den Schwefel an das Metall. Es handelt sich somit um chemisorbierte Disulfidfilme.

Die langen unverzweigten Alkylketten der erfindungsgemäßen Disulfide bewirken innerhalb der Disulfidmonoschicht ein zweidimensionales Kristallisieren. Diese Kristallisation verleiht dem Film größere thermische und mechanische Stabilität und macht ihn dichter gegenüber der Diffusion kleiner Teilchen durch den Film. Dichte monomolekulare Disulfidfilme stellen Korrosionsinhibitoren dar, wie durch Elektrochemische-Impedanz-Spektroskopie(EIS) gezeigt wurde. Die zweidimensionale Kristallisation, bedingt durch lange unverzweigte Alkylketten der Disulfide, erhöht zudem den Ordnungsgrad innerhalb der monomolekularen Schicht. Ein hoher Ordnungsgrad bewirkt die Exposition der endständigen funktionellen Gruppen des Disulfidfilmes an der Oberfläche. Hierdurch läßt sich durch Wahl geeigneter funktioneller Gruppen die Benetzbarkeit der Oberfläche gezielt modifizieren. Die funktionellen Gruppen können ferner für den gezielten chemischen Aufbau weiterer Schichten benutzt werden.

Bezüglich der erfindungsgemäßen Disulfide, deren Herstellung und Verwendung, ist im einzelnen folgendes auszuführen.

Die erfindungsgemäßen Disulfide der allgemeinen Formel (I)

$$X^1\text{-}(CH_2)_n\text{-}S\text{-}S\text{-}(CH_2)_n\text{-}X^2 \qquad \text{(I)},$$

enthalten langkettige Alkylenreste mit n = 11 bis n = 25, vorzugsweise n = 11 bis n = 13; sofern $X^1$ und $X^2$ untereinander gleich sind, stehen sie für die Substituenten

-Br,
-N-Phthalimid,
$-NH_2$,
$-OOC-(CH_2)_3-COOH$,
$-OSO_2-CH_3$,
$-NH-(CH_2)_2-NH_2$,
$-SO_3H$ oder
$-SO_3^-M^+$ mit $M^+ = Li^+$, $Na^+$ oder $K^+$;

$X^1$ und $X^2$ können jedoch auch untereinander verschieden sein und dann für -Br und -OH stehen;

Beispiele für Reste $-(CH_2)_n-$ sind

Undecandiyl
Dodecandiyl
Tridecandiyl
Tetradecandiyl
Pentadecandiyl
Hexadecandiyl
Heptadecandiyl
Octadecandiyl
Nonadecandiyl
Eicosandiyl
Tetracosandiyl und
Pentacosandiyl,

wobei Undecandiyl, Dodecandiyl und Tridecandiyl bevorzugt sind.

Die Herstellung der erfindungsgemäßen Disulfide der allgemeinen Formel (I) kann für den Fall, daß $X^1$ und $X^2$ untereinander gleich sind und für -Br, $-OOC-(CH_2)_3-COOH$, $-OSO_2-CH_3$ sowie für den Fall, daß $X^1$ für -OH und $X^2$ für -Br stehen, dadurch erfolgen, daß in einem ersten Syntheseschritt ein entsprechender endständig bromsubstituierter Alkohol der Formel $Br-(CH_2)_n-OH$ mit n = 11 bis 25 mit der äquimolaren Menge, oder einem geringen Überschuß Natriumthiosulfat in Wasser oder einem polaren organischen Lösungsmittel, wie z.B. Methanol, Ethanol, Isopropanol, Butanol oder Ethylenglykol und deren Gemische mit Wasser bei Temperaturen von 60 bis 120°C, vorzugsweise bei der Siedetemperatur des verwendeten Lösungsmittels, zum Buntesalz umgesetzt wird, dessen Lösung, beispielsweise in siedendem Ethanol, in einem zweiten Syntheseschritt mit Iod zum Disulfid oxidiert wird. In einem dritten Syntheseschritt wird dann das Disulfid für den Fall $X^1 = X^2$ = -Br und für den Fall $X^1$ = -OH und $X^2$ = -Br bei 60 bis 120°C mit Bromwasserstoff behandelt, beispielsweise mit wäßriger Bromwasserstofflösung unter Rückfluß gerührt, wobei sowohl das Monosubstitutionsprodukt ($X^1$ = -OH, $X^2$ = -Br) als auch das Disubstitutionsprodukt ($X^2 = X^2$ =-Br) gebildet werden. Die so erhältlichen unterschiedlich substituierten Disulfide lassen sich durch Säulenchromatographie (beispielsweise unter Verwendung von Silikagel) trennen und reinigen.

Für den Fall, daß $X^1$ und $X^2$ in der allgemeinen Formel (I) für $-OOC-(CH_2)_3-COOH$ stehen, wird das im zweiten Syntheseschritt erhaltene Produkt im dritten Syntheseschritt mit Glutarsäureanhydrid bei Temperaturen zwischen etwa 70 und 120°C, beispielsweise durch etwa einstündiges Rühren bei 90°C, zum entsprechenden Halbester der Glutarsäure umgesetzt, der gegebenenfalls durch Umkristallisieren weiter gereinigt werden kann.

Für den Fall, daß $X^1$ und $X^2$ in der allgemeinen Formel (I) für $-O-SO_2-CH_3$ stehen, wird das im zweiten Syntheseschritt erhaltene Disulfid in einem dritten Syntheseschritt bei Temperaturen zwischen 0 und 20°C, vorzugsweise bei 0°C in einem wasserfreien aprotischen organischen Lösungsmittel mit Methansulfonylchlorid in Gegenwart eines tertiären Amins, beispielsweise Triethylamin, umgesetzt. Die so erhaltene Lösung kann dann bei Raumtemperatur noch zwei Stunden gerührt werden, das entstandene Aminhydrochlorid wird dann durch Filtration entfernt, das Filtrat kann dann mit der gleichen Menge Wasser ausgeschüttelt werden. Aus der organischen Phase erhält man nach Verdampfen des Lösungsmittels das erfindungsgemäße Disulfid mit $X^1 = X^2 = -O-SO_3-CH_3$ als feste Substanz, die noch durch Umkristallisieren gereinigt werden kann.

Für die Herstellung erfindungsgemäßer Disulfide mit $X^1 = X^2 = -NH-CH_2-CH_2-NH_2$ kann man das Disulfid mit $X^1 = X^2 = -O-SO_2-CH_3$ mit überschüssigem Ethylendiamin bei Temperaturen zwischen 50 und 100°C umsetzen, beispielsweise durch etwa dreistündiges Rühren der ethanolischen Lösung unter Rückfluß. Anschließend kann das Ethanol im Rotationsverdampfer entfernt, der Rückstand in Dichlormethan aufgenommen und zweimal mit Wasser ausgeschüttelt werden. Aus der organischen Phase erhält man dann das Disulfid mit $-NH-CH_2-CH_2-NH_2$ Endgruppen, das durch Umkristallisieren, beispielsweise aus Hexan, gereinigt werden kann.

Zur Herstellung erfindungsgemäßer Disulfide mit $X^1 = X^2$ = -N-Phthalimid wird in einem ersten Syntheseschritt ein endständig bromsubstituierter Alkohol der Formel $Br-(CH_2)_n-OH$ mit n = 11 bis 25 mit der äquimolaren Menge Kaliumphthalimid in einem polaren aprotischen organischen Lösungsmittel, z.B. Dimethylformamid, bei Temperaturen zwischen 50 und 100°C umgesetzt, beispielsweise durch dreistündiges Rühren bei 70°C. Das Reaktionsprodukt wird durch Eingießen seiner Lösung in die doppelte Menge Wasser ausgefällt und kann durch Umkristallisieren aus einem Heptan/Ethanolgemisch weiter gereinigt werden. Das so erhaltene Produkt kann in einem zweiten Syntheseschritt mit einem Überschuß an konzentrierter wäßriger Bromwasserstofflösung, beispielsweise der dreifachen Menge 47 %iger wäßriger Bromwasserstofflösung, und konzentrierter Schwefelsäure, beispielsweise einer äquimolaren Menge konzentrierter Schwefelsäure, bei Temperaturen zwischen 60 und 120°C, beispielsweise durch vierstündiges Erhitzen unter Rückfluß, behandelt werden. Das beim Abkühlen auf Raumtemperatur ausfallende Produkt kann durch Umkristallisieren, z.B. aus Hexan, weiter gereinigt werden. Das so erhaltene Produkt kann dann in einem dritten Syntheseschritt in einem protischen organischen Lösungsmittel, beispielsweise in siedendem Ethanol, gelöst mit der äquimolaren Menge, oder einem geringen Überschuß, Natriumthiosulfat umgesetzt und in Wasser oder einem polaren organischen Lösungsmittel oder dessen Gemischen mit Wasser, beispielsweise in einem Gemisch aus Wasser und Ethanol, auf Temperaturen zwischen 60 bis 120°C, beispielsweise durch sechsstündiges Erhitzen am Rückfluß, erhitzt werden. Das so erhaltene Produkt kann durch Umkristallisieren, z.B. aus Ethanol, weiter gereinigt werden. In einem vierten Syntheseschritt kann dieses Produkt in einem protischen organischen Lösungsmittel, beispielsweise in siedendem Ethanol, gelöst und mit der halbmolaren Menge Iod oxidiert, sowie gegebenenfalls durch Umkristallisieren gereinigt werden. Nach Beendigung der Iodzugabe kann zweckmäßigerweise noch ca. 15 Minuten unter Rückfluß gerührt, mit Natriumcarbonat neutralisiert und eine Iodfärbung durch Zusatz von Natriumdisulfit entfernt werden. Das bei Raumtemperatur kristallisierende Produkt kann aus einem Gemisch aus Heptan und Ethylacetat umkristallisiert werden.

Zur Herstellung erfindungsgemäßer Disulfide der allgemeinen Formel (I) mit $X^1 = X^2 = NH_2$ wird das nach vorstehendem Verfahren hergestellte Disulfid mit -N-Phthalimidendgruppen mit überschüssigem Hydrazin in einem polaren organischen Lösungsmittel, z.B. Ethanol, bei Raumtemperatur, z.B. unter fünfstündigem Rühren der Reaktionsmischung, behandelt. Der dabei entstandene Niederschlag wird durch Zugabe von wäßriger Alkalilauge, z.B. 10 %ige Natronlauge, in Lösung gebracht und das Produkt mit Ether extrahiert. Das nach Verdampfen des Ethers zurückbleibende Produkt kann durch Umkristallisieren aus Hexan weiter gereinigt werden.

Zur Herstellung der erfindungsgemäßen Disulfide der allgemeinen Formel (I) mit $X^1 = X^2 = -SO_3Na$ wird in einem ersten Syntheseschritt ein endständig bromsubstituierter Alkohol der Formel $Br-(CH_2)_n-OH$ mit n = 11 bis 25 mit der in Wasser gelösten äquimolaren Menge Natriumsulfit bei Temperaturen zwischen 80 und 120°C umgesetzt, beispielsweise durch zehnstündiges Erhitzen unter Rückfluß. Das beim Abkühlen der Reaktionslösung anfallende Produkt kann zur weiteren Reinigung aus Wasser umkristallisiert werden. In einem zweiten Syntheseschritt wird dieses Produkt mit einem Überschuß an wäßriger konzentrierter Bromwasserstofflösung und konzentrierter Schwefelsäure, vorzugsweise der äquimolaren Menge, auf Temperaturen zwischen 60 und 120°C, z.B. fünf Stunden am Rückfluß, erhitzt. Das beim Abkühlen ausfallende Produkt kann zur weiteren Reinigung aus Ethanol umkristallisiert werden. Dieses Produkt wird

in einem dritten Syntheseschritt mit der äquimolaren Menge, oder einem geringfügigen Überschuß, Natriumthiosulfat in Wasser bei Temperaturen zwischen 80 und 120°C z.B. durch fünfstündiges Erhitzen unter Rückfluß, umgesetzt. Zur siedenden Lösung dieses Umsetzungsproduktes wird die halbmolare Menge Iod, gelöst in einem polaren organischen Lösungsmittel, wie z.B. Ethanol, zugegeben, anschließend mit Natriumcarbonat neutralisiert und das beim Abkühlen ausfallende Produkt gegebenenfalls aus Wasser umkristallisiert.

Durch kurzes Aufkochen des erfindungsgemäßen Disulfids der allgemeinen Formel (I) mit $X^1 = X^2 = -SO_3Na$ in überschüssiger konzentrierter Mineralsäure, z.B. Salzsäure, erhält man beim Abkühlen Disulfide der allgemeinen Formel (I) mit $X^1 = X^2 = -SO_3H$, die aus Wasser umkristallisiert werden können.

Wie bereits oben erwähnt, eignen sich die erfindungsgemäßen Disulfide sehr vorteilhaft zur Herstellung chemisorbierter monomolekularer Schichten auf Edelmetalloberflächen.

Als Träger für monomolekulare Schichten bestehend aus Disulfiden sind Edelmetalloberflächen wie beispielsweise Gold-, Silber-, Platin- oder Palladiumoberflächen geeignet, wobei auch ultradünne Überzüge dieser Metalle - bestehend aus wenigen Atomlagen - auf anderen Trägermaterialien für den Aufbau der Disulfidschicht ausreicht. Wichtig für den Aufbau einer Disulfidschicht ist die Bildung einer kovalenten oder koordinativen Bindung des Disulfides an Metallatome der Trägeroberfläche. Die Herstellung der Disulfidschichten erfolgt vorzugsweise durch Eintauchen des Träger in Lösungen der Disulfide und anschließendes Abspülen des überschüssigen nicht chemisorbierten Disulfidmaterials mit reinem Lösungsmittel. Durch Eintauchen in Lösungen von Gemischen mehrerer Disulfide können Disulfidmischfilme hergestellt werden. Anstatt die Träger in Disulfidlösungen zu tauchen, können die Träger auch mit Disulfidlösungen besprüht werden oder auf eine andere Weise mit den Disulfiden in Kontakt gebracht werden. Das Verfahren zum Aufbau der Disulfidschichten ist unabhängig von der Art des Lösungsmittels. Das Lösungsmittel sollte lediglich eine ausreichende Löslichkeit der Disulfide gewährleisten.

Die erfindungsgemäß hergestellten monomolekularen Disulfidschichten (1. Schicht) weisen an der Oberfläche exponierte funktionelle Gruppen auf. Diese funktionellen Gruppen ermöglichen durch chemische Reaktion den Aufbau einer 2. Schicht. Sofern die 2. Schicht an der Oberfläche wiederum funktionelle Gruppen aufweist, kann durch chemische Reaktion eine 3. Schicht aufgebaut werden. Nach diesem Verfahrensprinzip sind multimolekulare Schichten herstellbar. Die Anzahl der Schichten ist prinzipiell nicht begrenzt.

Erfindungsgemäß hergestellte multimolekulare Schichten (Multischichten) bedeuten zwei oder mehrere monomolekulare Schichten verschiedener Materialien, die chemisch d. h. kovalent, ionisch oder koordinativ miteinander verbunden sind.

Der Multischichtaufbau erfolgt Schicht für Schicht durch aufeinander folgendes Tauchen in Lösungen der schichtbildenden Materialien. Zwischen den Tauchvorgängen wird zweckmäßigerweise jeweils mit reinem Lösungsmittel nachgespült, um überschüssiges schichtbildendes Material zu entfernen.

Der einfachste Multischichtaufbau entspricht also dem Typ ABABAB..., aber durch Verwendung von mehr als zwei schichtbildenen Materialien sind auch komplexere Multischichtsysteme wie beispielsweise vom Typ ABCDCBABC... oder ABCBCDC... herstellbar, wobei A funktionelle Gruppen enthält, die mit B reagieren, B funktionelle Gruppen enthält, die mit A und C reagieren, C funktionelle Gruppen enthält, die mit B und D reagieren und D funktionelle Gruppen enthält, die mit C reagieren.

Bei der Wahl der funktionellen Gruppen ist wichtig, daß die funktionellen Gruppen der folgenden Schicht mit den funktionellen Gruppen der vorangegangenen Schicht chemisch reagieren und durch die Reaktion eine chemische Bindung z.B. kovalenter, ionischer oder koordinativer Natur gebildet wird. Dies gewährleistet eine gute und dauerhafte Haftung der Multischichtsysteme, hohe thermische und mechanische Stabilität sowie hohe Resistenz gegenüber Lösungsmitteln und Chemikalien.

Zum Aufbau von Multischichten mit 3 oder mehr Schichten werden als schichtbildende Materialien di- oder polyfunktionelle Substanzen benötigt, da eine funktionelle Gruppe mit der vorherigen Schicht und eine weitere funktionelle Gruppe mit der nachfolgenden Schicht reagiert und auf diese Weise die chemische Bindung der einzelnen Schichten aneinander erfolgt.

Die Verwendung von di- oder polyfunktionellen Substanzen zum Aufbau von Multischichten mit 3 oder mehr Schichten kann trickreich vermieden werden, indem monofunktionelle cyclische Substanzen verwendet werden, die durch Reaktion mit der vorherigen Schicht gleichzeitig durch eine Ringöffnungsreaktion eine neue funktionelle Gruppe erzeugen. Diese neue funktionelle Gruppe kann mit der nachfolgenden Schicht reagieren. Als monofunktionelle cyclische Substanzen sind besonders cyclische Anhydride, wie beispielsweise Glutarsäure-, Bernsteinsäure- und Maleinsäureanhydrid, Sultone, wie 1,3-Propansulton, 1,4-Butansulton etc. und cyclische reaktive Ether, Thioether und Amine wie Ethylenoxid, Ethylensulfid und Ethylenamin geeignet.

Sofern die funktionellen Gruppen der vorherigen Schicht nicht spontan mit der nachfolgenden Schicht reagieren, können sie durch geeignete Chemikalien aktiviert werden. So können beispielsweise Carboxylgruppen mittels Thionylchlorid, Oxalsäuredichlorid, Carbodiimiden und dergleichen zur Reaktion mit nukleophilen Gruppen der nachfolgenden Schicht aktiviert werden.

1) Mögliche funktionelle Gruppen zum Aufbau der 2. Schicht:
Die funktionellen Gruppen der 1. Schicht sind durch die Verwendung der erfindungsgemäßen Disulfide vorgegeben.

1a) Wenn die 1. Schicht (Disulfidschicht) endständige nukleophile Gruppen aufweist ($X^1$ oder $X^1$ = $-NH_2$, $-NH-(CH_2)_2-NH_2$, -OH) eignen sich zum Aufbau der 2. Schicht Substanzen, die elektrophile Gruppen tragen, wie beispielsweise: Anhydridgruppen, Säurechloridgruppen, Aldehydgruppen, Alkylhalogenidgruppen, Sulfonsäureestergruppen, Schwefelsäureestergruppen, Isocyanatgruppen, Isothiocyanatgruppen, Acrylatgruppen, Acrylamidgruppen, Maleinimidgruppen, Vinylsulfonylgruppen, Epoxidgruppen und ähnliche Gruppen.

1b) Wenn die 1. Schicht endständige basische Gruppen, beispielsweise Aminogruppen aufweist ($X^1$ oder $X^2$ = $-NH_2$, $-NH-(CH_2)_2-NH_2$) eignen sich zum Aufbau der 2. Schicht Substanzen, die Säuregruppen enthalten wie beispielsweise HOOC-, $HO_3S$-, $HO_3SO$-, $H_2O_3P$-und $H_2O_3PO$-Gruppen.

1c) Wenn die 1. Schicht endständige elektrophile Gruppen aufweist ($X^1$ oder $X^2$ = -Br, $-OSO_2-CH_3$) eignen sich zum Aufbau der 2. Schicht Substanzen, die nukleophile Gruppen haben wie beispielsweise Amminogruppen, Hydroxylgruppen, Mercaptogruppen, Carbaniongruppen oder Phosphingruppen.

1d) Wenn die 1. Schicht endständige $HO_3S$-Gruppen oder $HOOC-(CH_2)_3-COO$-Gruppen aufweist eignen sich zum Aufbau der 2. Schicht Substanzen, die basische Gruppen wie beispielsweise Aminogruppen, Amidingruppen, Guanidingruppen oder S-Alkylthioharnstoffgruppen aufweisen. Oder wenn die $HO_3S$-Gruppen bzw. $HOOC-(CH_2)_3-COO$-Gruppen mit Thionylchlorid, Oxalsäuredichlorid, Carbodiimiden und dergleichen aktiviert werden, eignen sich zum Aufbau der zweiten Schicht nukleophile Gruppen wie sie unter 1c) genannt sind.

1e) Wenn die 1. Schicht endständige $NaO_3S$-Gruppen aufweist eignen sich zum Aufbau der 2. Schicht Substanzen, die kationische Gruppen aufweist, wie beispielsweise Ammoniumgruppen, Amidiniumgruppen, Guanidiniumgruppen, Isothiuroniumgruppen und Phosphoniumgruppen.

2) Mögliche funktionelle Gruppen zum Aufbau der 3. und weiterer Schichten:

2a) Weist die vorherige Schicht endständig nukleophile Gruppen auf, so eignen sich für den Aufbau der folgenden Schicht Substanzen mit elektrophilen Gruppen, wie beispielsweise unter 1a) genannt.

2b) Weist die vorherige Schicht endständige basiche Gruppen auf, wie beispielsweise Aminogruppen, Amidingruppen, Guanidingruppen, S-Alkylthioharnstoffgruppen, eignen sich zum Aufbau der folgenden Schicht Substanzen, die Säuregruppen enthalten wie beispielsweise HOOC-, $HO_3S$-, $HO_3SO$-, $H_2O_3P$- und $H_2O_3PO$-Gruppen.

2c) Wenn die vorherige Schicht endständige elektrophile Gruppen aufweist, eignen sich zum Aufbau der 2. Schicht Substanzen, die nukleophile Gruppen haben, wie beispielsweise Aminogruppen, Hydroxylgruppen, Mercaptogruppen, Carbaniongruppen und Phosphingruppen.

2d) Weist die vorherige Schicht Säuregruppen auf wie beispielsweise HOOC-, $HO_3S$-, $HO_3SO$-, $H_2O_3P$-und $H_2O_3PO$-Gruppen, so eignen sich zum Aufbau der folgenden Schicht Substanzen mit basischen Gruppen wie beispielsweise Aminogruppen, Amidingruppen, Guanidingruppen und S-Alkylthioharnstoff-gruppen.

Neben ihrer Verwendung als 1. Schicht für einen nachfolgenden Multischichtaufbau dienen monomolekulare Disulfidschichten als ultradünne Gleitschichten etwa für magnetische Aufzeichnungsmedien, ferner als ultradünne Korrosionsschutzschichten von Silber- bzw. Goldoberflächen.

Durch den erfindungsgemäßen Aufbau multimolekularer Schichten auf Elektroden, Schwingquarzen, Oberflächenwellenbauteilen oder optischen Bauteilen lassen sich deren Eigenschaften maßschneidern. Beispielsweise können eine oder mehrere Schichten des Multischichtaufbaus Rezeptoren für Biomoleküle darstellen und somit für die Biosensorik verwendet werden. Schichten, die Chemorezeptoren (Kronenether, Kryptanden) enthalten, finden Anwendung in der Chemosensorik, vorallem in der Gassensorik und Ionensensorik.

Multischichtsysteme, die Chromophore enthalten, sind durch Nutzung nichtlinearer optischer Effekte in der Lage als optische Schalter oder Frequenzverdoppler zu wirken.

Die in den Beispielen angegebenen Teile und Prozente sind, soweit nicht anders angegeben, Gewichtsteile bzw. Gewichtsprozente.

Beispiel 1

a) Herstellung von Verbindung (1) gemäß Formelschema

$$HO\text{-}(CH_2)_{11}\text{-}Br + Na_2S_2O_3 \rightarrow HO\text{-}(CH_2)_{11}\text{-}S\text{-}SO_3Na \quad (1)$$

50 g (0,199 mol) 11-Bromundecansäure und 49,4 g (0,199 mol) Natriumthiosulfatpentahydrat werden mit 200 g Wasser und 160 g Ethanol versetzt und 4 Stunden unter Rückfluß erhitzt. Während des Abkühlens fällt das Rohprodukt kristallin aus. Man saugt ab, trocknet das Rohprodukt im Vakuum und kristallisiert aus 880 g Ethanol um. Die Ausbeute beträgt 48,3 g (81 %) farblose Kristalle.
Identifizierung: DC (Dünnschichtchromatographie); IR- und $^1$H-NMR-Spektrum; Schmelzpunkt: 181-183°C.
b) Herstellung von Verbindung (2) gemäß Formelschema

$$2\ HO\text{-}(CH_2)_{11}\text{-}S\text{-}SO_3Na + I_2 \rightarrow [HO\text{-}(CH_2)_{11}\text{-}S]_2 \quad (2)$$

31,5 g (103 mmol) der Verbindung (1) werden mit 84 g Ethanol und 50 g Wasser versetzt. Man erwärmt das Gemisch bis zum Sieden und tropft langsam eine Lösung von 13,05 g (51,4 mmol) Jod in 116 g Ethanol zu. Anschließend wird mit Natriumcarbonat neutralisiert und die Jodfärbung der Lösung durch Zugabe von wässriger Natriumdisulfitlösung entfärbt. Bein Abkühlen scheidet sich das Rohprodukt kristallin ab. Man saugt ab und kristallisiert aus 80 g Methanol um.
Ausbeute: 17,9 g (86 %) farblose Kristalle.
Identifizierung: DC; IR- und $^1$H-NMR-Spektrum;
Schmelzpunkt: 79-81°C

| Elementaranalyse: | | | |
|---|---|---|---|
| ber. | C 64.97 % | H 11.40 % | S 15.77 %; |
| gef. | C 64.90 % | H 11.30 % | S 15.80 % |

c) Herstellung der Verbindung (3) und der erfindungsgemäßen Verbindung (4) gemäß Formelschema

$$[HO\text{-}(CH_2)_{11}\text{-}S]_2$$

$$+\ HBr \rightarrow [Br\text{-}(CH_2)_{11}\text{-}S]_2 + HO\text{-}(CH_2)_{11}\text{-}S\text{-}S\text{-}(CH_2)_{11}\text{-}Br$$

(3) (4)

2 g (4.92 mmol) der Verbindung (2), 3.81 g (14.76 mmol) 47 prozentige wässrige Bromwasserstofflösung und 0.62 g (6.32 mmol) konzentrierte Schwefelsäure werden 5 Stunden unter Rückfluß erhitzt. Im Dünnschichtchromatogramm (= DC) ist kein Edukt mehr nachweisbar. Bei Raumtemperatur scheiden sich braune Kristalle ab. Nach Absaugen, Waschen mit Wasser und Trocknen erhält man 2.38 g (91%) Rohprodukt. Das Rohprodukt wird säulenchromatographisch an Kieselgel (40-63 µm) mit Hexan:Ethylacetat = 1:1 gereinigt. Dabei erhält man 1.56 g (60%) gelbliche Kristalle, die nach zweimaligem Umkristallisieren aus Pentan mit 0.92 g (35%) Ausbeute als farbloser kristalliner Feststoff erhalten werden.
Nach IR- und $^1$H-NMR-Spektrum handelt es sich um Verbindung (3) des obigen Formelschemas. Die zweite Fraktion der Säulenchromatographie (0.22 g kristalliner Feststoff) wird ebenfalls aus Pentan umkristallisiert und man erhält 0.18 g (7 %) Ausbeute farbloser Kristalle. Nach IR- und $^1$H-NMR-Spektrum handelt es sich um Verbindung (4).
Identifizierung von Verbindung (3):
Schmelzpunkt: 34°C; DC; IR- und $^1$H-NMR-spektrum
Identifizierung von Verbindung (4):
Schmelzpunkt: 55°C; DC; IR- und $^1$H-NMR-Spektrum

Beispiel 2

Herstellung der erfindungsgemäßen Verbindung (5) gemäß Formelschema

$$[HO-(CH_2)_{11}-S]_2 + \text{Glutarsäureanhydrid} \rightarrow [HO_2C-(CH_2)_3CO_2-(CH_2)_{11}-S]_2 \quad (5)$$

5 g (12.3 mmol) der Verbindung (2) und 2.95 g (25.85 mmol) Glutarsäureanhydrid werden 1 Stunde bei 90 C gerührt. Im DC ist kein Edukt mehr nachweisbar. Beim Erkalten kristallisiert das Rohprodukt. Es wird zweimal aus einem Gemisch Heptan : Aceton = 8 : 2 umkristallisiert. Man erhält 6.61 g (84%) farblose Kristalle.
Identifizierung: DC; IR- und $^1$H-NMR-Spektrum; Schmelzpunkt: 79-80°C;

| Elementaranalyse: | | | | |
|---|---|---|---|---|
| ber. | C 60.53 % | H 9.21 % | O 20.16 % | S 10.10 %; |
| gef. | C 60.60 % | H 9.30 % | O 20.30 % | S 10.10 % |

Beispiel 3

Herstellung der erfindungsgemäßen Verbindung (6) gemäß Formelschema

$$[HO-(CH_2)_{11}-S]_2 + Cl-SO_2-CH_3 \rightarrow [H_3C-SO_3-(CH_2)_{11}-S]_2 \quad (6)$$

5.0 g (12.3 mmol) der Verbindung (2) und 2.74 g (27.0 mmol) trockenes Triethylamin werden in 27 g trockenem Dichlormethan gelöst und auf 0 C gekühlt. Dann werden 2.95 g (25.7 mmol) Methansulfonylchlorid, in 4 g trockenem Dichlormethan gelöst, langsam zugetropft. Nach Beendigung des Zutropfens läßt man den Ansatz auf Raumtemperatur kommen und rührt 2 Stunden bei Raumtemperatur. Es wird über Kieselgel filtriert, das Filtrat mit Ethylacetat verdünnt, zweimal mit Wasser ausgeschüttelt und im Scheidetrichter getrennt. Die organische Phase wird über Natriumsulfat getrocknet und die Lösungsmittel werden am Rotationsverdampfer entfernt. Der Rückstand wird zweimal aus einem Gemisch Heptan : Ethylacetat = 7 : 3 umkristallisiert. Man erhält 4.35 g (63%) farblose Kristalle.
Identifizierung: DC; IR- und $^1$H-NMR-Spektrum; Schmelzpunkt: 69-73°C.

Beispiel 4

Herstellung der erfindungsgemäßen Verbindung (7) gemäß Formelschema

$$[H_3C-SO_3-(CH_2)_{11}-S]_2 + H_2N-(CH_2)_2-NH_2 \rightarrow$$

$$[H_2N-(CH_2)_2-NH-(CH_2)_{11}-S]_2 \quad (7)$$

2.0 g (3.55 mmol) der Verbindung (6), 2.45 g (40.7 mmol) Ethylendiamin und 8 g Ethanol werden 3 Stunden unter Rückfluß erhitzt. Im DC ist kein Edukt mehr nachweisbar. Man läßt abkühlen, gibt 0.4 g Natriumhydroxid zu, rührt 30 Minuten bei Raumtemperatur und schüttelt dreimal mit je 40 g Dichlormethan aus. Die organische Phase wird abgetrennt, über Natriumsulfat getrocknet und das Dichlormethan am Rotationsverdampfer entfernt. Man erhält 1.3 g Feststoff, der nach Umkristallisieren aus Hexan mit 0.87 g (51%) Ausbeute als farbloser kristalliner Feststoff erhalten wird.
Identifizierung: DC; IR- und $^1$H-NMR-Spektrum; Schmelzpunkt: 68-74°C.

Verbindung (7) hat die besondere Eigenschaft Schwermetallionen wie beispielweise Cu, Cd, Hg, Ni, Pb, Zn, Pd, Rh und andere zu binden. Aufgrund der guten Löslichkeit der Komplexverbindungen von (7) mit diesen Schwermetallen in organischen Lösungsmitteln wie beispielsweise Ethylacetat, Chloroform, Dichlormethan, Diethylether und anderen eignet sich Verbindung (7) um die genannten Schwermetalle aus wässriger Lösung in die organische Phase zu extrahieren. Desweiteren eignen sich monomolekulare Schichten von (7) auf Sensoroberflächen um die genannten Schwermetallionen zu dedektieren. Mit (7) können sogar reversible Sensoren hergestellt werden, da durch Zugabe von Säure und anschließende Zugabe von Lauge Verbindung (7) aus seinen Schwermetallkomplexen regeneriert werden kann.

Beispiel 5

a) Herstellung der Verbindung (8) gemäß Formelschema

$$HO\text{-}(CH_2)_{11}\text{-}Br + \text{Kaliumphthalimid} \rightarrow$$

$$HO\text{-}(CH_2)_{11}\text{-}N\text{-}\text{Phthalimid} \qquad (8)$$

24.33 g (0.131 mol) Kaliumphthalimid werden in 236 g Dimethylformamid suspendiert. 30.0 g (0.119 mol) 11-Bromundecanol werden zugefügt und 3 Stunden bei 70°C gerührt. Man läßt abkühlen und gießt in 600 g Wasser, wobei das Rohprodukt ausfällt. Nach Absaugen und Trocknen wird aus 270 g eines Gemisches von Heptan : Ethanol = 8 : 2 umkristallisiert. Man erhält 33.2 g (88 %) farblose Kristalle.
Identifizierung: DC; IR- und $^1$H-NMR-Spektrum ; Schmelzpunkt: 82-84°C.

b) Herstellung der Verbindung (9) gemäß Formelschema

$$HO\text{-}(CH_2)_{11}\text{-}N\text{-}\text{Phthalimid} + HBr \rightarrow Br\text{-}(CH_2)_{11}\text{-}N\text{-}\text{Phthalimid} \qquad (9)$$

10.0 g (31 mmol) der Verbindung (8) werden mit 24 g (93mmol) 47 prozentiger wässriger Bromwasserstoffsäure und 3.0 g (31 mmol) konzentrierter Schwefelsäure versetzt. Man erhitzt 4 Stunden unter Rückfluß und läßt über Nacht bei Raumtemperatur stehen. Die wässrige saure Lösung wird von den ausgefallenen Kristallen abpipettiert und die Kristalle werden mit Wasser säurefrei gewaschen. Nach Trocknen und Umkristallisieren aus Hexan erhält man 10.0 g (85%) kristallinen Feststoff.
Identifizierung: DC; IR- und $^1$H-NMR-Spektrum; Schmelzpunkt: 54°C.

c) Herstellung der Verbindung (10) gemäß Formelschema

$$Br\text{-}(CH_2)_{11}\text{-}N\text{-}\text{Phthalimid} + Na_2S_2O_3 \rightarrow$$

$$NaO_3S\text{-}S\text{-}(CH_2)_{11}\text{-}N\text{-}\text{Phthalimid} \qquad (10)$$

20.0 g (52.6 mmol) der Verbindung (9), 13.05 g (52.6 mmol) Natriumthiosulfatpentahydrat, 65 g Wasser und 52 g Ethanol werden 6 Stunden unter Rückfluß erhitzt. Im DC ist kein Edukt mehr erkennbar. Man läßt abkühlen, saugt ab, kristallisiert aus Ethanol um und erhält 16.9 g (74 %) farblose Kristalle.
Identifizierung: DC; IR- und $^1$H-NMR-Spektrum; Schmelzpunkt: 127-129°C.

d) Herstellung der erfindungsgemäßen Verbindung (11) gemäß Formelschema

$$2\ NaO_3S\text{-}S\text{-}(CH_2)_{11}\text{-}N\text{-}\text{Phthalimid} + I_2 \rightarrow$$

$$[\text{Phthalimid-N-}(CH_2)_{11}\text{-}S\text{-}]_2 \qquad (11)$$

11.45 g (26.3 mmol) der Verbindung (10) werden in 28 g Ethanol und 35 g Wasser gelöst und zum Sieden erhitzt. In die siedende Lösung werden 3.5 g (13.8 mmol) Iod, gelöst in 20 g Ethanol, eingetropft und nach Beendigung des Zutropfens noch 15 Minuten gerührt. Man neutralisiert mit Natriumcarbonat, läßt abkühlen und entfernt die Iodfärbung mit wenig wässriger Natriumdisulfitlösung. Bei Raumtemperatur kristallisiert das Rohprodukt über Nacht aus. Es wird abgesaugt, mit Wasser gewaschen, getrocknet und aus einem Gemisch Heptan : Ethylacetat = 8 : 2 umkristallisiert. Man erhält 6.7 g (77 %) farblose Kristalle.
Identifizierung: DC; IR- und $^1$H-NMR-Spektrum; Schmelzpunkt: 60-62°C;

| Elementaranalyse: | | | | |
|---|---|---|---|---|
| ber. | C 68.64 % | H 7.88 % | N 4.21 % | S 9.64; |
| gef. | C 68.90 % | H 8.00 % | N 4.40 % | S 9.50 % |

Beispiel 6

Herstellung der erfindungsgemäßen Verbindung (12) gemäß Formelschema

$$[\text{Phthalimid-N-}(CH_2)_{11}\text{-S-}]_2 + N_2H_4 \rightarrow [H_2N\text{-}(CH_2)_{11}\text{-S-}]_2 \qquad (12)$$

1.0 g (1.5 mmol) der Verbindung (11) werden in 8 g Ethanol gelöst und mit 1.5 g (30 mmol) Hydrazinhydrat versetzt. Man rührt 5 Stunden bei Raumtemperatur, wobei ein Niederschlag entsteht. Im DC ist kein Edukt mehr nachweisbar. Man gibt 1,5 g NaOH, gelöst in 15 g Wasser zu, worauf sich der Niederschlag löst, extrahiert mit Ether und kristallisiert das aus der Etherphase erhaltene Produkt aus 8 g Hexan um. Man erhält 0.38 g (62%) farblose Kristalle.
Identifizierung: DC; IR- und $^1$H-NMR-Spektrum; Schmelzpunkt: 69-72°C.

Beispiel 7:

a) Herstellung der Verbindung (13) gemäß Formelschema

$$HO\text{-}(CH_2)_{11}\text{-Br} + Na_2SO_3 \rightarrow HO\text{- }(CH_3)_{11}\text{-}SO_3Na \qquad (13)$$

25.1 g (0.1 mol) 11-Bromundecanol, und 13.9 g (0.11 mol) Natriumsulfit in 50 g Wasser werden 10 Stunden unter Rückfluß erhitzt. Beim Abkühlen auf Raumtemperatur kristallisiert das Produkt aus. Man saugt ab, rührt das Produkt 1 Stunde in 72 g Ether, saugt erneut ab und kristallisiert aus Wasser um. Nach Trocknen bei 60°C im Vakuum erhält man 19.2 g (70 %) farblose Kristalle.
Identifizierung: DC; IR- und $^1$H-NMR-Spektrum; Schmelzpunkt: 230-235°C

b) Herstellung der Verbindung (14) gemäß Formelschema

$$HO\text{-}(CH_2)_{11}\text{-}SO_3Na + HBr \rightarrow Br\text{- }(CH_2)_{11}\text{-}SO_3H \qquad (14)$$

16.0 g (58.3 mmol) der Verbindung (13) werden mit 40.15 g (233.2 mmol) 47 prozentiger wässriger Bromwasserstofflösung und 5.83 g (58.3 mmol) konzentrierter Schwefelsäure versetzt und 5 Stunden unter Rückfluß erhitzt, wobei der Ansatz dunkel wird. Beim Abkühlen kristallisiert das Rohprodukt aus. Umkristallisieren aus 70 g Wasser ergibt 9.5 g (52%) kristallinen Feststoff, der dunkle Verunreinigungen enthält. Nochmaliges Umkristallisieren aus 360 g Ethanol ergibt 7.5 g (41%) farblose Kristalle.
Identifizierung: DC; IR- und $^1$H-NMR-Spektrum; Schmelzpunkt: 127°C (Zersetzung)

c) Herstellung der erfindungsgemäßen Verbindung (15) gemäß Formelschema

$$Br\text{-}(CH_2)_{11}\text{-}SO_3H + Na_2S_2O_3 \rightarrow NaO_3S\text{-S-}(CH_2)_{11}\text{-}SO_3Na;$$

$$2\ NaO_3S\text{-S-}(CH_2)_{11}\text{-}SO_3Na + I_2 \rightarrow [NaO_3S\text{-}(CH_2)_{11}\text{-S-}]_2 \qquad (15)$$

5.0 g (15.8 mmol) der Verbindung (14) und 4.12 g (16.6 mmol) Natriumthiosulfatpentahydrat werden in 30 g Wasser 5 Stunden unter Rückfluß erhitzt. Im DC ist kein Edukt mehr erkennbar. Dann tropft man in die siedende Lösung eine Lösung von 2.3 g (9.13 mmol) Iod in Ethanol und rührt nach Beendigung des Zutropfens 15 Minuten. Man neutralisiert mit Natriumcarbonat, läßt abkühlen und entfärbt die Lösung mit Natriumdisulfit. Beim Abkühlen fällt das Produkt aus. Umkristallisieren aus 35 g eines Gemisches von Ethanol und Wasser im Verhältnis 3 zu 1 ergibt 2.85 g (67%) farblose Kristalle.
Identifizierung: DC; IR-Spektrum; Schmelzpunkt: 240°C (Zersetzung)

Beispiel 8

Herstellung der erfindungsgemäßen Verbindung (16) gemäß Formelschema

$$[NaO_3S\text{-}(CH_2)_{11}\text{-}S\text{-}]_2 + HCl \rightarrow [HO_3S\text{-}(CH_2)_{11}\text{-}S\text{-}]_2 \qquad (16)$$

2.0 g (3.45 mmol) der Verbindung (15) werden in 60 g konzentrierter Salzsäure kurz aufgekocht. Der beim Abkühlen ausfallende Niederschlag wird abgesaugt, mit kaltem Ethanol gewaschen und aus einem Gemisch von Wasser und Ethanol im Verhältnis 1 : 1 umkristallisiert.
Identifizierung: DC; IR- und $^1$H-NMR-Spektrum

Beispiel 9

1) Herstellung der Träger

Auf frisch gespaltenen Glimmer wird im Hochvakuum eine etwa 5 nm dicke Chromschicht aufgedampft um die Haftung der nachfolgenden Edelmetallschicht zu verbessern. Anschließend wird eine etwa 100 nm dicke Gold- oder Silberschicht aufgedampft. Anstelle des Glimmers können auch andere Substrate wie beispielsweise Siliziumwafer, Glas, Quarzglas etc. verwendet werden. Die auf diese Weise hergestellten Träger werden im folgenden Gold- oder Silberträger genannt, unabhängig vom Substratmaterial, welches unter der Gold- oder Silberschicht ist. Die durch den Aufdampfvorgang erhaltenen Edelmetallschichten sind polykristallin. Sobald die frisch hergestellten Gold- oder Silberträger mit Luft in Berührung kommen, adsorbieren sie hydrophobe Verunreinigungen aus der Luft. Dies zeigt sich an der Zunahme des Kontaktwinkels gegenüber einem Wassertropfen, bis sich bei einem Kontaktwinkel von 85-90° ein konstanter Endwert einstellt. Die hydrophoben Verunreinugungen behindern die Chemisorption der erfindungsgemäßen Disulfide nicht. Die hohe Affinität des Schwefels der Disulfide zum Gold und Silber bewirkt eine Verdrängung der Verunreinigungen von der Gold- bzw. Silberoberfläche.

2) Herstellung der Tauchbadlösungen

Die Art des Lösungsmittels ist für die Chemisorption der Disulfide nicht ausschlaggebend. Das Lösungsmittel soll lediglich eine ausreichende Löslichkeit der Disulfide gewährleisten. Sehr polare Disulfide wie die Verbindungen (15) und (16) werden in Wasser gelöst, polare Disulfide wie die Verbindungen (12), (7) und (5) werden in Ethanol gelöst und unpolare Disulfide wie die Verbindungen (3), (4), (6) und (11) werden in Ethylacetat oder Heptan gelöst. Die Konzentration der Disulfidlösungen kann in weitem Bereich variiert werden, ohne die Chemisorption der Disulfide auf die Gold- oder Silberträger negativ zu beeinflussen. Im Konzentrationsbereich von $10^{-2}$ bis $10^{-4}$ Mol pro Liter werden chemisorbierte Disulfidschichten von gleichbleibender Qualität erhalten.

3) Herstellung der monomolekularen Disulfidschichten

Gold- bzw. Silberträger werden in Tauchbadlösungen der Disulfide getaucht, wobei spontan Chemisorption der Disulfide an die Gold- bzw. Silberoberfläche erfolgt. Tauchzeiten von ungefähr 17 Stunden ergeben sehr dichte Disulfidschichten mit hoher molekularer Ordnung innerhalb der Schicht. Längere Tauchzeiten schaden nicht. Tauchzeiten von 5 bis 15 Minuten ergeben auch chemisorbierte Disulfidschichten, die Oberflächenbelegung ist aber nicht in allen Fällen vollständig. Sogar bei einer Tauchzeit von nur 1 Minute sind chemisorbierte Disulfidschichten auf Gold- bzw. Silberträgern durch Kontaktwinkeländerungen nachweisbar.

Nach dem Tauchvorgang werden die beschichteten Gold- bzw. Silberträger aus dem Tauchbad herausgeholt und mit reinem Lösungsmittel gespült, um überschüssiges Disulfidmaterial, das nicht an die Gold- bzw. Silberoberfläche chemisorbiert ist, zu entfernen. Das Spülen gewährleistet, daß lediglich eine monomolekulare, chemisch fest gebundene Disulfidschicht auf dem Gold- bzw. Silberträger zurückbleibt.

4) Identifizierung der monomolekularen Disulfidschichten

Kontaktwinkelmessungen:
Änderungen im Kontaktwinkel beschichteter gegenüber unbeschichteter Gold- bzw. Silberträger zeigen das Vorhandensein einer Disulfidschicht an. Disulfide mit endständigen polaren Gruppen erzeugen auf den Trägern hydrophile Oberflächen (kleine Kontaktwinkel). Disulfide mit endständigen unpolaren Gruppen erzeugen hydrophobe Oberflächen (große Kontaktwinkel). Dies zeigt deutlich, daß die monomolekularen Disulfidschichten die Benetzungseigenschaften der Träger modifizieren. So können durch einfaches Tauchen hydrophobe, oleophobe und hydrophile Gold- bzw. Silberoberflächen hergestellt werden. Die Ergebnisse sind im folgenden tabellarisch zusammengefaßt.

| | Disulfid | Kontaktwinkel ($H_2O$) | |
|---|---|---|---|
| | | Gold | Silber |
| (11) | [Phthalimid-N-$(CH_2)_{11}$-S-$]_2$ | 74° | 73° |
| (12) | [$H_2N$-$(CH_2)_{11}$-S-$]_2$ | 64° | 67° |
| (4) | Br-$(CH_2)_{11}$-S-S-$(CH_2)_{11}$-OH | 61° | 71° |
| (7) | [$H_2N$-$(CH_2)_2$-NH-$(CH_2)_{11}$-S-$]_2$ | 59° | |
| (6) | [$H_3C$-$SO_3$-$(CH_2)_{11}$-S-$]_2$ | 55° | 65° |
| (5) | [$HO_2C$-$(CH_2)_3$-$CO_2$-$(CH_2)_{11}$-S-$]_2$ | 44° | 42° |
| (15) | [$NaO_3S$-$(CH_2)_{11}$-S-$]_2$ | < 10° | < 10° |

Grazing-Incidence-IR-Spektroskopie (GIR):

GIR-Spektren von chemisorbierten monomolekularen Schichten der erfindungsgemäßen Disulfide auf Gold- bzw. Silberträgern zeigen die typischen Schwingungen für $CH_2$-Gruppen bei 2919 und 2849 $cm^{-1}$.

Die Disulfidschichten sind thermisch außerordentlich stabil. Thermische Belastung bis 150°C zeigt keinerlei Desorption oder Zerstörung der Schichten.

Elektrochemische-Impedanz-Spektroskopie (EIS):

EIS-Spektren von chemisorbierten monomolekularen Schichten der erfindungsgemäßen Disulfide auf Gold- bzw. Silberträgern zeigen, daß die Schichten sehr dicht, und vorallem gegenüber wässrigen Elektrolyten undurchlässig sind. Demnach sind die Schichten porenfrei und wirken als Korrosionsinhibitoren. Desweiteren sind sie gegenüber wässrigen Elektrolyten stabil, d. h. es findet im Laufe der Zeit keine Ablösung oder Zerstörung der Disulfidschichten statt.

## Patentansprüche

**1.** Disulfide der allgemeinen Formel (I)

$$X^1\text{-}(CH_2)_n\text{-S-S-}(CH_2)_n\text{-}X^2 \qquad (I),$$

worin $X^1$ und $X^2$ untereinander gleich sind und für die Substituenten
-N-Phthalimid, -$NH_2$, -OOC-$(CH_2)_3$-COOH, -$OSO_2$-$CH_3$,
-NH-$(CH_2)_2$-$NH_2$, -$SO_3H$ oder -$SO_3^-M^+$ mit $M^+$ = $Li^+$, $Na^+$ oder $K^+$ stehen und n für eine ganze Zahl von 11 bis 25 steht,
oder $X^1$ für -OH und $X^2$ für -Br stehen und n für 11 bis 25 steht,

**2.** Disulfide nach Anspruch 1, dadurch gekennzeichnet, daß in der allgemeinen Formel (I) n = 11 bis 13 bedeutet.

**3.** Verfahren zur Herstellung der Disulfide der allgemeinen Formel (I) $X^1$-$(CH_2)_n$-S-S-$(CH_2)_n$-$X^2$, worin die Substituenten $X^1$ und $X^2$ untereinander gleich sind und für -OOC-$(CH_2)_3$-COOH oder -$OSO_2$-$CH_3$ stehen oder $X^1$ für -OH und $X^2$ für -Br steht, dadurch gekennzeichnet, daß im ersten Syntheseschritt ein endständig bromsubstituierter Alkohol der Formel Br-$(CH_2)_n$-OH mit n = 11 bis 25 mit der äquimolaren Menge Natriumthiosulfat in Wasser oder einem polaren organischen Lösungsmittel bei Temperaturen von 60 bis 120°C zum Buntesalz umgesetzt wird, im zweiten Syntheseschritt die Lösung des Buntesalzes mit Iod zum Disulfid oxidiert wird, im dritten Syntheseschritt das Disulfid mit Bromwasserstoff bei 60 bis 120°C behandelt wird, wobei sowohl das Monosubstitutionsprodukt ($X^1$ = -OH, $X^2$ = -Br) als auch das Disubstitutionsprodukt ($X^1$ = $X^2$ = -Br) gebildet wird, und die beiden unterschiedlich substituierten Disulfide getrennt werden, wobei für den Fall, daß $X^1$ und $X^2$ in der Formel (I) für -OOC-$(CH_2)_3$-COOH stehen, das Produkt des 2. Syntheseschrittes im dritten Syntheseschritt mit Glutarsäureanhydrid bei 70 bis 120°C zum entsprechenden Halbester der Glutarsäure umgesetzt wird und gegebenenfalls anschließend durch Kristallisation gereinigt wird, für den Fall, daß $X^1$ und $X^2$ in der Formel (I) für -$OSO_2$-$CH_3$ stehen, das Produkt des 2. Syntheseschrittes im dritten Syntheseschritt bei Temperaturen zwishen 0 bis 20°C in einem wasserfreien aprotischen organischen Lösungsmittel mit Methansulfonylchlorid in Gegenwart eines tertiären Amins umgesetzt wird, das entstandene Aminhydrochlorid entfernt, und aus der verbleibenden Lösung nach Verdampfen des Lösungsmittels das Disulfind mit

$$X^1 = X^2 = \text{-O-SO}_2\text{-CH}_3$$

erhalten wird.

**4.** Verfahren zur Herstellung der Disulfide der allgemeinen Formel (I) gemäß Anspruch 1, worin die Substituenten $X^1$ und $X^2$ untereinander gleich sind und für $-NH-(CH_2)_2-NH_2$ stehen, dadurch gekennzeichnet, daß das gemäß Anspruch 3 hergestellte Disulfid mit $X^1 = X^2 = -O-SO_2-CH_3$ mit überschüssigem Ethylendiamin bei 50 bis 100°C umgesetzt und gegebenenfalls gereinigt wird.

**5.** Verfahren zur Herstellung der Disulfide der allgemeinen Formel (I) gemäß Anspruch 1, worin die Substituenten $X^1$ und $X^2$ untereinander gleich sind und für -N-Phthalimid stehen, dadurch gekennzeichnet, daß in einem ersten Syntheseschritt ein endständig bromsubstituierter Alkohol der Formel $Br-(CH_2)_n-OH$ mit n = 11 bis 25 mit der äquimolaren Menge Kaliumphthalimid in einem polaren aprotischen Lösungsmittel bei 50 bis 100°C umgesetzt, das Reaktionsprodukt durch Ausfällen in Wasser erhalten und gegebenenfalls durch Umkristallisieren gereinigt wird, das so erhaltene Produkt in einem zweiten Syntheseschritt mit einem Überschuß an konzentrierter wäßriger Bromwasserstofflösung und konzentrierter Schwefelsäure auf Temperaturen zwischen 60 und 120°C erhitzt, das bei Raumtemperatur ausfallende Produkt, gegebenenfalls durch Umkristallisieren gereinigt, in einem dritten Syntheseschritt mit der äquimolaren Menge Natriumthiosulfat versetzt und in Wasser oder einem polaren organischen Lösungsmittel oder dessen Gemisch mit Wasser auf Temperaturen von 60 bis 120°C erhitzt, das so erhaltene Produkt, gegebenenfalls durch Umkristallisieren gereinigt, in einem vierten Syntheseschritt in einem protischen organischen Lösungsmittel gelöst und mit Iod oxidiert und gegebenenfalls durch Umkristallisieren gereinigt wird.

**6.** Verfahren zur Herstellung der Disulfide der allgemeinen Formel (I) gemäß Anspruch 1, worin die Substituenten $X^1$ und $X^2$ untereinander gleich sind und für $-NH_2$ stehen, dadurch gekennzeichnet, daß das gemäß dem Verfahren nach Anspruch 5 hergestellte Produkt mit überschüssigem Hydrazin in einem polaren organischen Lösungsmittel bei Raumtemperatur behandelt wird, der dabei entstandene Niederschlag durch Zugabe von wäßriger Alkalilauge in Lösung gebracht, das Produkt mit Ether extrahiert und das nach Verdampfen des Ethers erhaltene Rohprodukt gegebenenfalls durch Umkristallisieren gereinigt wird.

**7.** Verfahren zur Herstellung der Disulfide der allgemeinen Formel (I) gemäß Anspruch 1, worin die Substituenten $X^1$ und $X^2$ untereinander gleich sind und für $-SO_3^-M^+$, z.B. $-SO_3^-Na^+$, stehen, dadurch gekennzeichnet, daß in einem ersten Syntheseschritt ein endständig bromsubstituierter Alkohol der Formel $Br-(CH_2)_n-OH$ mit n = 11 bis 25 mit der in Wasser gelösten äquimolaren Menge Natriumsulfit bei Temperaturen zwishen 80 und 120°C umgesetzt, das beim Abkühlen anfallende, gegebenenfalls umkristallisierte, Produkt in einem zweiten Syntheseschritt mit einem Überschuß einer wäßrigen konzentrierten Bromwasserstofflösung und konzentrierter Schwefelsäure auf Temperaturen zwischen 60 und 120°C erhitzt, das beim Abkühlen ausfallende, gegebenenfalls umkristallisierte, Produkt in einem dritten Syntheseschritt mit der äquimolaren Menge Natriumthiosulfat in Wasser bei Temperaturen zwischen 80 und 120°C umgesetzt wird, zur siedenden Lösung dieses Umsetzungsproduktes die halbmolare Menge Iod, gelöst in einem polaren organischen Lösungsmittel, getropft, anschließend mit Natriumcarbonat neutralisiert und das beim Abkühlen ausfallende Produkt gegebenenfalls aus Wasser umkristallisiert wird.

**8.** Verfahren zur Herstellung der Disulfide der allgemeinen Formel (I) gemäß Anspruch 1, worin die Substituenten $X^1$ und $X^2$ untereinander gleich sind und für $-SO_3H$ stehen, dadurch gekennzeichnet, daß das gemäß dem Verfahren nach Anspruch 7 hergestellte Produkt in überschüssiger konzentrierter Mineralsäure aufgekocht und der beim Abkühlen ausfallende Feststoff gegebenenfalls umkristallisiert wird.

**9.** Verfahren zur Herstellung monomolekularer Schichten auf Edelmetallträgern mit Disulfiden gemäß Anspruch 1 oder 2, dadurch gekennzeichnet, daß der Edelmetallträger mit einer Lösung des Disulfides in Kontakt gebracht wird, wodurch eine Chemisorption des Disulfides auf dem Edelmetallträger erfolgt.

**10.** Verfahren zur Herstellung von multimolekularen Schichten auf Trägern, deren Oberfläche durch Aufbringen von Disulfidschichten nach dem Verfahren gemäß Anspruch 9 modifiziert ist, dadurch gekennzeichnet, daß der Aufbau der multimolekularen Schichten sequenziell, d.h. Schicht für Schicht, erfolgt und die einzelnen Schichten miteinander chemisch verbunden sind.

## Claims

1. A disulfide of the formula (I)

$$X^1\text{-}(CH_2)_n\text{-S-S-}(CH_2)_n\text{-}X^2 \qquad (1)$$

where $X^1$ and $X^2$ are identical to one another and are each -N-phthalimide, $-NH_2$, -OOC- $(CH_2)_3$-COOH, $-OSO_2$-$CH_3$, $-NH(CH_2)_2-NH_2$, $-SO_3H$ or $-SO_3^-M^+$ in which $M^+$ is $Li^+$, $Na^+$ or $K^+$, and n is an integer from 11 to 25, or $X^1$ is -OH and $X^2$ is -Br and n is from 11 to 25.

2. A disulfide as claimed in claim 1, wherein, in the formula (I), n is from 11 to 13.

3. A process for the preparation of a disulfide of the formula (I), $X^1\text{-}(CH_2)_n\text{-S-S-}(CH_2)_n\text{-}X^2$, where $X^1$ and $X^2$ are identical to one another and are each $-OOC-(CH_2)_3-COOH$ or $-OSO_2-CH_3$ or $X^1$ is -OH and $X^2$ is -Br, wherein, in a first synthesis step, a terminally bromine-substituted alcohol of the formula $Br-(CH_2)_n-OH$, where n is from 11 to 25, is reacted with an equimolar amount of sodium thiosulfate in water or in a polar organic solvent at from 60 to 120°C to give the Bunte salt, in the second synthesis step the solution of the Bunte salt is oxidized with iodine to the disulfide, in the third synthesis step the disulfide is treated with hydrogen bromide at from 60 to 120°C, both the monosubstitution product ($X^1$ = -OH, $X^2$ = -Br) and the disubstitution product ($X^1$ = $X^2$ = -Br) being formed, and the two differently substituted disulfides are separated, where $X^1$ and $X^2$ in the formula (I) are each $-OOC-(CH_2)_3$-COOH the product of the second synthesis step being reacted in the third synthesis step with glutaric anhydride at from 70 to 120°C to give the corresponding half ester of glutaric acid and, if necessary, then being purified by crystallization, and where $X^1$ and $X^2$ in the formula (I) are each $-OSO_2-CH_3$ the product of the second synthesis step being reacted in the third synthesis step with methanesulfonyl chloride at from 0 to 20°C in an anhydrous aprotic organic solvent in the presence of a tertiary amine, the resulting amine hydrochloride being removed and the disulfide in which $X^1$ and $X^2$ are each $-O-SO_2-CH_3$ being obtained from the remaining solution after evaporation of the solvent.

4. A process for the preparation of a disulfide of the formula (I) as claimed in claim 1, where $X^1$ and $X^2$ are identical to one another and are each $-NH-(CH_2)_2-NH_2$, wherein the disulfide prepared according to claim 3 in which $X^1$ and $X^2$ are each $-O-SO_2-CH_3$ is reacted with excess ethylenediamine at from 50 to 100°C and the product is, if required, purified.

5. A process for the preparation of a disulfide of the formula (I) as claimed in claim 1, where $X^1$ and $X^2$ are identical to one another and are each -N-phthalimide, wherein, in a first synthesis step, a terminally bromine-substituted alcohol of the formula $Br-(CH_2)_n-OH$ in which n is from 11 to 25 is reacted with an equimolar amount of potassium phthalimide in a polar aprotic solvent at from 50 to 100°C, the reaction product is obtained by precipitation in water and if necessary purified by recrystallization, the product thus obtained is heated in a second synthesis step with an excess of concentrated aqueous hydrogen bromide solution and concentrated sulfuric acid to 60-120°C, and the product precipitated at room temperature is, if required, purified by recrystallization, in a third synthesis step an equimolar amount of sodium thiosulfate is added and the mixture is heated in water or in a polar organic solvent or a mixture thereof to 60-120°C, and the product thus obtained is, if required, purified by recrystallization and, in a fourth synthesis step, is dissolved in a protic organic solvent and is oxidized with iodine and, if required, purified by recrystallization.

6. A process for the preparation of a disulfide of the formula (I) as claimed in claim 1, where $X^1$ and $X^2$ are identical to one another and are each $-NH_2$, wherein the product prepared by the process as claimed in claim 5 is treated with excess hydrazine in a polar organic solvent at room temperature, the resulting precipitate is brought into solution by adding aqueous alkali metal hydroxide solution, the product is extracted with ether and the crude product obtained after evaporation of the ether is, if required, purified by recrystallization.

7. A process for the preparation of a disulfide of the formula (I) as claimed in claim 1, where $X^1$ and $X^2$ are identical to one another and are each $-SO_3^-M^+$, eg. $-SO_3^-Na^+$, wherein, in a first synthesis step, a terminally bromine-substituted alcohol of the formula $Br-(CH_2)_n-OH$ in which n is from 11 to 25 is reacted with an equimolar amount of sodium sulfite, dissolved in water, at from 80 to 120°C, in a second synthesis step the product obtained on cooling, which may have been recrystallized, is heated with an excess of an aqueous concentrated hydrogen

bromide solution and concentrated sulfuric acid to 60-120°C, and in a third synthesis step the product precipitated on cooling, which may have been recrystallized, is reacted with an equimolar amount of sodium thiosulfate in water at from 80 to 120°C, the semimolar amount of iodine, dissolved in a polar organic solvent, is added dropwise to the boiling solution of this reaction product, neutralization is then effected with sodium carbonate and the product precipitated on cooling is, if required, recrystallized from water.

**8.** A process for the preparation of a disulfide of the formula (I) as claimed in claim 1, where $X^1$ and $X^2$ are identical to one another and are each $-SO_3H$, wherein the product prepared by the process as claimed in claim 7 is boiled in excess concentrated mineral acid and the solid precipitated on cooling is, if required, recrystallized.

**9.** A process for the production of a monomolecular layer on a noble metal substrate using a disulfide as claimed in claim 1 or 2, wherein the noble metal substrate is brought into contact with a solution of the disulfide, with the result that the disulfide is chemisorbed on the noble metal substrate.

**10.** A process for the production of a multimolecular layer on a substrate whose surface has been modified by applying a disulfide layer by the process as claimed in claim 9, wherein the multimolecular layer is built up sequentially, ie. layer by layer, and the individual layers are chemically bonded to one another.

**Revendications**

**1.** Disulfures de formule générale I

$$X^1\text{-}(CH_2)_n\text{-S-S-}(CH_2)_n\text{-}X^2 \qquad (I),$$

dans laquelle $X^1$ et $X^2$, ayant des significations identiques, représentent des substituants -N-phtalimide, $-NH_2$, $-OOC-(CH_2)_3-COOH$, $-OSO_2-CH_3$, $-NH-(CH_2)_2-NH_2$, $-SO_3H$ ou $-SO_3M^+$ dans lequel $M^+ = Li^+$, $Na^+$ ou $K^+$, et n est un nombre entier allant de 11 à 25,
ou bien $X^1$ représente -OH et $X^2$ représente -Br et n est un nombre allant de 11 à 25.

**2.** Disulfures selon la revendication 1, caractérisés par le fait que, dans la formule générale I, n est un nombre allant de 11 à 13.

**3.** Procédé de préparation des disulfures de formule générale I, $X1\text{-}(CH_2)_n\text{-}5\text{-S-}(CH_2)_n\text{-}X^2$, dans laquelle $X^1$ et $X^2$, ayant des significations identiques, représentent $-OOC-(CH_2)_3-COOH$ ou $-OSO_2-CH_3$ ou bien $X^1$ représente -OH et $X^2$ représente -Br, caractérisé par le fait que, dans le premier stade de synthèse, on convertit un alcool portant un substituant bromo en position terminale et répondant à la formule $Br-(CH_2)_n-OH$ dans laquelle n est un nombre allant de 11 à 25, par réaction avec la quantité équimoléculaire de thiosulfate de sodium, dans l'eau ou dans un solvant organique polaire, à des températures de 60 à 120°C, en sel de Bunte, au deuxième stade opératoire, on oxyde la solution du sel de Bunte en disulfure, à l'aide d'iode, au troisième stade de synthèse, on traite le disulfure par le bromure d'hydrogène à des températures de 60 à 120°C, ce qui donne à la fois le produit mono-substitué ($X^1$ = -OH, $X^2$ = -Br) et le produit disubstitué ($X^1 = X^2$ = -Br), qu'on sépare l'un de l'autre, et, dans le cas où $X^1$ et $X^2$ de la formule I représentent $-OOC-(CH_2)_3-COOH$, on convertit le produit du deuxième stade de synthèse par l'anhydride glutarique à des températures de 70 à 120°C, au troisième stade de synthèse, en l'hémi-ester correspondant de l'acide glutarique et, le cas échéant, on purifie ensuite par cristallisation ; dans le cas où $X^1$ et $X^2$ de la formule I représentent $-OSO_2-CH_3$, on fait réagir le produit du deuxième stade de synthèse à des températures de 0 à 20°C dans un solvant organique aprotonique anhydre, au troisième de synthèse, avec le chlorure de méthanesulfonyle en présence d'une amine tertiaire, on élimine le chlorhydrate d'amine formé et, à partir de la solution résiduelle, on isole par évaporation du solvant le disulfure pour lequel $X^1 = X^2 = -O-SO_2-CH_3$.

**4.** Procédé de préparation des disulfures de formule générale I selon la revendication 1 dans laquelle les symboles $X^1$ et $X^2$ sont identiques et représentent $-NH-(CH_2)_2-NH_2$, caractérisé par le fait que l'on fait réagir- le disulfure pour lequel $X^1 = X^2 = -O-SO_2-CH_3$, préparé selon la revendication 3, avec un excès d'éthylènediamine à des températures de 50 à 100°C et le cas échéant on purifie.

**5.** Procédé de préparation des disulfures de formule générale I selon la revendication 1 pour lesquels les substituants

$X^1$ et $X^2$ sont identiques et consistent en groupes -N-phtalimides, caractérisé par le fait que, dans un premier stade de synthèse, on fait réagir un alcool portant un substituant bromo en position terminale et répondant à la formule $Br-(CH_2)_n-OH$ dans laquelle n est un nombre allant de 11 à 25 avec la quantité équimoléculaire de phtalimide potassique dans un solvant aprotonique polaire à des températures de 50 à 100°C, on isole le produit de réaction par précipitation dans l'eau et, le cas échéant, on le purifie par recristallisation, on chauffe le produit obtenu dans un deuxième stade de synthèse avec un excès d'une solution aqueuse concentrée de bromure d'hydrogène et de l'acide sulfurique concentré à des températures de 60 à 120°C, on purifie le produit qui précipite à température ambiante le cas échéant par recristallisation, on ajoute, dans un troisième stade de synthèse, la quantité équimoléculaire de thiosulfate de sodium et on chauffe à des température de 60 à 120°C dans l'eau ou dans un solvant organique polaire ou un mélange d'un tel solvant et d'eau, on purifie le produit obtenu le cas échéant par recristallisation et, dans un quatrième stade de synthèse, on dissout dans un solvant organique protonique et on oxyde par l'iode et, le cas échéant, on purifie par recristallisation.

6. Procédé de préparation des disulfures de formule générale I selon la revendication 1 dans laquelle $X^1$ et $X^2$ sont identiques et représentent $-NH_2$, caractérisé par le fait que l'on traite le produit préparé par le procédé de la revendication 5 par un excès d'hydrazine dans un solvant organique polaire à température ambiante, on redissout le précipité formé par addition d'une lessive alcaline, on extrait le produit par l'éther et, le cas échéant, on purifie par recristallisation le produit brut isolé après vaporisation de l'éther.

7. Procédé de préparation des disulfures de formule générale I selon la revendication 1 dans laquelle $X^1$ et $X^2$ sont identiques et représentent $-SO_3M^+$, par exemple $-SO_3Na^+$, caractérisé par le fait que, dans un premier stade de synthèse, on fait réagir un alcool portant un substituant bromo en position terminale et répondant à la formule $Br-(CH_2)_n-OH$ dans laquelle n est un nombre allant de 11 à 25 avec la quantité équimoléculaire de sulfite de sodium en solution dans l'eau, à des températures de 80 à 120°C, dans un deuxième stade de synthèse, on chauffe le produit qui a précipité au refroidissement et qu'on a éventuellement recristallisé avec un excès d'une solution aqueuse concentrée de bromure d'hydrogène et de l'acide sulfurique concentré à des température de 60 à 120°C, dans un troisième stade de synthèse, on fait réagir le produit qui a précipité au refroidissement et qu'on a éventuellement recristallisé avec la quantité équimoléculaire de thiosulfate de sodium dans l'eau, à des températures de 80 à 120°C, on ajoute goutte à goutte à la solution bouillante de ce produit de réaction la quantité semi-molaire d'iode, en solution dans un solvant organique polaire, on neutralise ensuite par du carbonate de sodium et, le cas échéant, on recristallise dans l'eau le produit qui a précipité au refroidissement.

8. Procédé de préparation des disulfures de formule générale I selon la revendication 1 dans laquelle les symboles $X^1$ et $X^2$ ont des significations identiques et représentent $-SO_3H$, caractérisé par le fait que l'on soumet le produit préparé par le procédé selon la revendication 7 à ébullition dans un excès d'un acide minéral concentré et, le cas échéant, on recristallise la matière solide qui a précipité au refroidissement.

9. Procédé pour l'application de couches monomoléculaires sur des supports de métaux nobles à l'aide des disulfures selon la revendication 1 ou 2, caractérisé par le fait que l'on met le support de métal noble en contact avec une solution du disulfure, ce qui provoque une chimisorption du disulfure sur le support de métal noble.

10. Procédé pour l'application de couches multimoléculaires sur des supports dont la surface a été modifiée par application de couches de disulfures par le procédé selon la revendication 9, caractérisé par le fait que les couches multimoléculaires sont appliquées en séquence, c'est-à-dire couche par couche, et par le fait que les couches individuelles sont reliées chimiquement entre elles.